# EUROPEAN PATENT SPECIFICATION

(11) **EP 0 927 042 B1**
(45) Date of publication and mention of the grant of the patent: **11.02.2004**
(21) Application number: 97943048.5
(22) Date of filing: 19.09.1997
(51) Int. Cl.: A61K 38/18, A61K 35/20, A61P 1/00

(54) **PREVENTION OF GASTROINTESTINAL DAMAGE**
VERMEIDUNG VON GASTROINTESTINALER SCHÄDIGUNG
PREVENTION DU DELABREMENT GASTRO-INTESTINAL

(30) Priority: 20.09.1996 GB 9619660
(43) Date of publication of application: 07.07.1999
(73) Proprietor: SHS International Ltd., Liverpool L7 9PT (GB)
(72) Inventor: JOHNSON, Wendy, Susan, Merseyside (GB); PLAYFORD, Raymond, John, Leicester LE2 1SA (GB)
(74) Representative: Atkinson, Peter Birch
(86) International application number: PCT/GB1997/002574
(87) International publication number: WO 1998/011910

(56) References cited:
- EP-A- 0 269 408
- EP-A- 0 367 447
- EP-A- 0 527 283
- EP-A- 0 619 370
- EP-A- 0 652 015
- WO-A-92/00994
- WO-A-92/18153
- WO-A-93/07891
- WO-A-93/14783
- WO-A-95/00155
- WO-A-95/29933
- WO-A-96/34614
- CHEMICAL ABSTRACTS, vol. 111, no. 1, 3 July 1989 Columbus, Ohio, US; abstract no. 1522u, S. SUZUKI: "HUMAN EPIDERMAL GROWTH FACTOR IN BREAST MILK, EARLY NEONATAL URINE, AND AMNIOTIC FLUID: SPECULATION ON THE SOURCE AND PHYSIOLOGICAL ROLE." page 152; XP002053998 & NAGOYA MED. J., vol. 33, no. 2, 1988, pages 91-110,
- CHEMICAL ABSTRACTS, vol. 103, no. 11, 16 September 1985 Columbus, Ohio, US; abstract no. 82348b, L. JANSSON ET AL.: "MITOGENIC ACTIVITY AND EPIDERMAL GROWTH FACTOR CONTENT IN HUMAN MILK." page 121; XP002053999 & ACTA PAEDIATR. SCAND., vol. 74, no. 2, 1985, pages 250-253,
- R.J. PLAYFORD ET AL.: "BOVINE COLOSTRUM IS PROPHYLACTIC AGAINST INDOMETHACIN-INDUCED INTESTINAL INJURY." GASTROENTEROLOGY, vol. 112, no. 4 SUPPL., April 1997, BALTIMORE, PA, US, page A394 XP002053545

## Description

The present invention relates to the prevention of gastrointestinal damage and more particularly, but not exclusively, to such damage which occurs in the intestine.

There are a variety of gastrointestinal conditions in which damage to epithelial type cells occur. For example, this damage may be in the form of ulceration, increased permeability with protein and blood loss from the intestine or structuring.

Gastrointestinal conditions involving damage to epithelial cells arise after prolonged administration of Non Steroidal Anti-inflammatory Drugs (NSAIDs) (e.g. indomethacin, ibuprofen, azapropazone, naproxen, piroxicam. ketoprofen, diclofenac, asprin etc) to patients who require protection from chronic inflammatory medical conditions. Examples of such chronic conditions which require prolonged administration of NSAIDs are rheumatoid arthritis and osteoarthritis. NSAID therapy is also beneficial for sufferers of Cystic Fibrosis (to ameliorate the inflammatory process causing lung damage). Long term use of NSAIDs is associated with a high risk of developing gastric or intestinal damage including structuring, fibrosis and ulceration. A major effect of NSAIDs is damage to gastrointestinal epithelial cells which may lead to the development of ulcers. Such ulcers may unexpectedly haemorrhage or become perforated. This leads to the requirement for emergency treatment and when undetected may even be associated with mortality. Mortality is especially associated with patients on NSAIDs who develop ulcers that become perforated because these patients are often symptomless and do not suffer pain because of the pain dampening effects of NSAIDs.

Up to 60% of NSAID-taking patients complain of dyspepsia or generalised abdominal discomfort. It has also been reported that gastric ulceration occurs in 12-30%, and duodenal lesions in 2-19%. of long term NSAID users. When it is considered that NSAIDs account for 5% of prescribed drugs in the UK but account for 25% of all reported adverse effects. it is apparent that these adverse effects associated with NSAIDs are a major problem and that a satisfactory means of preventing NSAID-induced damage to gastrointestinal epithelial cells would be of considerable benefit.

Various measures are used to treat NSAID-induced ulcers. These include the use of several types of pharmaceutical products. such as Histamine H₂ receptor antagonists, sucralfate, prostaglandins (e.g. misoprostol) and hydrogen-potassium pump inhibiting agents (e.g. omeprazole). A major disadvantage of these products is that, although they are effective at treating gastric damage, they are largely ineffective when directed towards intestinal epithelium. It is therefore desirable that there is an agent which is effective for intestinal epithelium. It is particularly desirable that there is a suitable agent that may be used prophylactically, to prevent gastrointestinal damage occurring. For instance, it would be of great beneficial to treat someone prophylactically if it is anticipated they will require NSAID therapy in order that gastrointestinal damage may be avoided. For example, prophylactic treatment would be of benefit for sufferers of rheumatoid arthritis, osteoarthritis or cystic fibrosis who are taking NSAIDs.

It is an object of the present invention to obviate or mitigate the above mentioned disadvantages and provide a prophylactic agent that may be used to prevent damage occurring to gastrointestinal epithelium as a result of administration of NSAIDs.

According to a first aspect of the present invention, there is prodded the use of a composition containing Transforming Growth Factor β for the manufacture of a medicament for prophylactic treatment, of a gastrointestinal condition at least partially characterised by damage to epithelial cells, the condition being caused by administration of a non-steroidal anti-inflammatory drug.

By "prophylactic treatment" we mean either (i) a treatment that protects gastrointestinal epithelium such that a gastrointestinal condition that is at least partially characterised by damage to epithelial cells does not occur; or (ii) a treatment that prevents healthy epithelial cells from becoming damaged if a gastrointestinal condition already exists.

The invention is applicable for prophylactic treatment of patients who are at risk of suffering NSAID-induced damage to gastrointestinal epithelial cells (such as sufferers of rheumatoid arthritis. osteoarthritis or cystic fibrosis who are taking NSAIDs). Therefore the composition containing TGF-β may be used to prophylactically prevent damage caused by NSAIDs such as indomethacin. ibuprofen, azapropazone, naproxen, piroxicam, ketoprofen, diclofenac or asprin.

We have found that compositions containing TGF-β are effective at prophylactically treating gastrointestinal conditions that are at least partially characterised by damage to epithelial cells as a result of NSAID administration. Examples of such compounds include naturally produced TGF-β isolated from the organism in which it was synthesised, TGF-β produced by cell culture or fermentation of TGF-β expressing cells, artificially synthesised TGF-β, modified TGF-β produced from genetically engineered organisms, compounds which activate TGF-β receptors (TGF-β receptor agonists), compounds which regulate TGF-β intracellular signalling and compounds which influence TGF-β synthesis or breakdown.

It is preferred that the composition containing TGF-β is a composition containing at least, one compound with activity similar to that of TGF-β obtainable from colostrum. We have found that the specific type and quantity of TGF-β obtainable from colostrum are particularly effective at preventing damage to gastrointestinal epithelial cells. A most preferred composition for use according to the invention is colostrum or a derivative thereof which contains viable TGF-β.

Colostrum is the milk secreted by the mammary gland during the first 48 hours following parturition. The composition of this ''first milk" is fundamentally different to that of the subsequently secreted normal milk. In particular, colostrum contains specific types and increased concentrations of growth factors. The colostrum used in the present invention is preferably the milk obtained in the first 2 milkings following parturition. Derivatives of such milk may also be used.

The particular effectiveness of colostrum or a derivative thereof is surprising because we have found that administration of Growth Factors provided by normal milk, or a derivative thereof to subject in need of prophylactic treatment is much less effective for preventing such damage than administration of Growth Factors obtainable from colostrum or a derivative thereof.

Compositions for use in the invention contain a transforming growth factor (e.g. TGFβ1, TGFβ2 or TGFβ3).

Our studies have established that members of the Transforming Growth Factor.β (TGFβ) family of growth factors are particularity effective for protecting gastrointestinal epithelium. It is therefore preferred that compositions used in accordance with the invention contain a TGFβ, e.g.TGFβ1. TGFβ2 and/or TGFβ3. Compositions containing said TGFβ may be derived from any source, although it is preferred that such compositions are derived from colostrum.

While we do not want to be bound by any hypothesis, it is possible that these compositions protect epithelial cells by regulating apoptosis or regulating modelling of the extracellular matrix in the gastrointestinal tract.

it is preferred that compositions used in accordance with the invention are to be administered at least once daily. Prophylactic treatment with compositions containing TGF-β may continue until the risk of gastrointestinal damage occuring has been removed. It is also preferred that the composition is to be administered from 6 to 72 hours (more preferably from 24 to 48) in advance of initiation of NSAID therapy and should ideally continue until NSAID therapy has stopped.

It will be appreciated that the amount of a composition containing TGF-β required for prophylactic treatment of a (NSAID induced) gastrointestinal condition that is at least partially characterised by damage to epithelial cells depends on a number of factors. These include:
A) The efficacy of TGF-β within the composition.
B) The amount of TGF-β within the composition.
C) The severity of the condition to be treated.
D) The age of the subject to be treated.

The amount of TGFβ used for prophylactic treatment, is preferably from 0.05 to 2500 µgrammes/day. It is preferred that from 25 to 1000 µgrammes/day of said TGFβ is to be administered to a subject in need of treatment and most preferred that from 50 to 500 µgrammes/day said TGFβ is to be admnistered.

Compositions containing TGF-β may be administered in various ways. Thus a medicament of the invention may take a number of different forms. For example, the medicament may be in the form of a powder, tablet, capsule, liquid, food product or drink product or any other suitable form that may be administered to a person or animal. Food products (such as confectionery bars) and drink products are particularly preferred. These products may also contain flavouring, carbohydrates, a nitrogen source, vegetable oils, emulsifiers, oils containing long chain fatty acids, antioxidants, vitamins, soluble fibre, minerals, other trace elements and the like to meet nutritional requirements and to make such products more palatable.

It will be appreciated that the vehicle for the composition should be one which is well tolerated by the subject to whom it is given and enables delivery of the active composition to the gastrointestinal tract.

The composition used according to the invention is preferably to be administered by an enteral route. However. TGF-β activity in certain compositions (containing growth factors) is lost when administered by an enteral route. We believe this is due to digestion by luminal proteases. For this reason it is particularly preferred that a composition for enteral administration is colostrum or a derivative thereof We have found that administration of TGF-β activity within colostrum, or a derivative thereof. surprisingly protects the compound contained therein from being degraded and thereby allows the delivery of active compounds to gastrointestinal epithelium.

The route of enteral administration may be by means of an enema, a nasogastric tube or alternatively by means of gastrostomy tubes or jejunostomy tubes. A most preferred route of enteral administration is by an oral route.

Use of colostrum, or a derivative thereof, for the manufacture of a medicament for prophylactic treatment of gastrointestinal conditions offers many advantages over therapies currency being used. For instance, large quantities of bovine colostrum can be readily obtained from dairy cattle. This means there are sufficient resources for the wide scale prophylactic use of compositions containing TGF-β that are derived from colostrum. Furthermore, bovine colostrum is a natural and safe resource.

We have established that, to provide sufficient TGF-β to have a protective effect on gastrointestinal epithelium, bovine colostral whey is to be preferably administered in the range of from 30 to 300mls/day. From 0.1 to 60.0 grammes/day represents a suitable daily dosage of spraydried derivatives of bovine colostrum. It will be appreciated that the amount of a colostrum derivative required will depend upon the precise extraction or purification steps undertaken to prepare such a colostrum derivative.

The colostrum used in the invention as a source of TGF-β is preferably bovine colostrum. Preferred colostrum derivatives are therefore derivatives of bovine colostrum.

As indicated earlier a colostrum derivative may be used in accordance with the invention. Such colostrum derivatives are those which contain the TGF-β that may be found in colostrum. Examples of preferred colostrum derivatives are:
1. Colostrum may be spraydried to form a powder before being used. If desired the spraydried derivative may be reconstituted in the form of a spraydried skimmed milk drink. The colostrum may be defatted before spray drying if desired.
2. Colostral whey or a derivative thereof. Colostral whey is colostrum from which casein proteins have been removed. Derivatives suitable for use according to the invention include ultrafiltered or microfiltered fractions of colostral whey. These fractions contain more concentrated Growth Factors relative to remaining colostral proteins and nutrients. Colostral whey may be used in liquid form (which may be defatted if desired) or may be further treated (such as being spraydried) before use according to the invention.

As stated above. a most preferred composition of the invention is bovine colostrum or a derivative thereof. The colostrum may be obtained by normal milking procedures, after which it may be pooled and frozen prior to being processed, if desired, to produce a colostrum derivative.

If desired, the colostrum (or derivative thereof) may be supplemented with one or more growth factors (e.g. purified growth factors) such as an IGF (e.g. IGF-1 or IGF-2), a transforming growth factor (e.g. TGFβ1. TGFβ2 or TGFβ3), a keratinocyte growth factor, a fibroblast growth factor, and/or a platelet-derived growth factor.

Colostrum, or a derivative thereof, may be formulated with other agents to form a composition suitable for enteral consumption. For instance, agents such as carbohydrates, a nitrogen source. vegetable oils. emulsifiers. oils containing long chain fatty acids, antioxidants, vitamins. soluble fibre or minerals and other trace elements may be included in the composition to fulfil nutritional requirements when colostrum is being incorporated into a food product (such as a confectionery bar) or drink product (in which case a liquid derivative or a powder derivative reconstituted as a liquid may be used). Alternatively derivatives of colostrum may be formulated with suitable excipients, stabilizers and the like to make a tablet, capsule or liquid medicament.

Additionally flavouring may also be included to make the composition more palatable.

The invention is illustrated by the following non-limiting example with reference to the accompanying drawings, in which:
Figure 1 represents the results of Example 1 for determining the effect of a colostrum derivative on indomethacin induced damage of gastrointestinal epithelium in mice; and
Figure 2 represents the results of Example 2 for determining the effect of TGFβ on indomethacin induced damage of gastrointestinal epithelium in rats.

### EXAMPLE 1

### METHODS

### 1. In vivo model of small intestinal injury

### Protocol:

Mice were randomised into groups of twenty and fed on a standard chow diet *ad libitum.* The drinking water was supplemented with 10% solution of defatted colostrum or milk whey for six days. Pilot studies showed that the addition of these solutions did not affect the total volume drunk (mean 5 ml/mouse/day). Small intestinal injury was induced in half of the animals in each group by administering a single dose of indomethacin (85mg/Kg sc.). Animals were killed 24 h later. In order to assess changes in proliferation, each animal also received vincristine (I mg/Kg i.p.) two hours prior to killing.

### Assessment of damage and proliferation:

The wet weight of the various sections of the gastrointestinal tract were recorded and samples of the small intestine and colon (defined by their percentage length) were fixed in Carnoy's fluid and stored in 70% (v/v) ethanol. Tissues were subsequently stained with the Feulgen reaction and the crypts displayed by microdissection. The numbers of arrested metaphases in 20 crypts per animal per site were counted.

Differences in villus height (as an index of intestinal damage) were determined in various regions of the intestine by microdissecting the tissue and tracing the outline of the villi using a stereo dissecting microscope. Tracings were subsequently scanned and analysed by computer image analyses.

### 2. In vitro model of cell proliferation.

Male August rats were anaesthetised and hepatocytes were isolated by *in situ* collagenase perfusion. The digested liver was removed, the cells dispersed, filtered, centrifuged and re-suspended in a plating-medium. For all studies. hepatocytes were grown in Williams E medium without L-glutamine containing 5% foetal calf serum.

Sixteen hours after plating, wells received various concentrations of Epidermal Growth Factor (EGF). EGF was used in these studies to stimulate hepatocyte proliferation. Each well also received 10% colostrum or vehicle (control) to assess the effect of colostrum on EGF stimulated growth. In addition. some wells also received TGFβ or a TGFβ neutralising antibody to examine the effect of TGFβ on cell growth.

The rate of proliferation was assessed by measuring the rate of hepatocyte DNA synthesis. [³H]Thymidine (2µCi/well) was added to each well twelve hours after the addition of EGF (and the other agents) and cells were left for a further 16h before measuring cellular [³H]thymidine incorporation. For each condition, the stimulatory or inhibitory effect of the solutions was measured in quadruplet in four separate wells. Cell viability, determined by the ability to exclude 0.2% trypan blue was greater than 80% in all experiments.

### 3. Statistics

Studies were assessed using a two-way ANOVA (with diet and presence of indomethacin as factors for the *in vivo* studies and test solution and presence of TGFβ neutralising antibody as factors for the *in vitro* studies).. Where a significant effect was seen (P < 0.05), individual comparisons between groups were performed based on the group means, residual and degrees of freedom obtained from the ANOVA.

### RESULTS

### In vivo studies

The *in vivo* model of damage to gastrointestinal epithelium allows accurate quantitation of the degree of gastrointestinal injury and is used to determine the protective effects of growth factors involved in mucosal integrity and repair. In these studies, maximal damage occurs 24 hours after administration of indomethacin. Colostrum treated animals have very little damage after this time. This indicates that growth factors in colostrum reduce the degree of initial damage rather than increasing the rate of repair.

Animals which received colostrum or milk solutions but had not been given indomethacin showed no significant changes in gastrointestinal epithelium proliferation or villus morphology compared to control animals.

Indomethacin caused a 25% reduction in the proliferation rate (as determined by 2-hour metaphase assessment. P < 0.001 ) of the small and large intestine in control animals and also those receiving colostrum or milk solution.

At both jejunal and ileal sites. indomethacin also caused a 25% reduction in the villus heights of control animals. Similar changes were seen in animals which had received 10% milk solution. Animals receiving 10% colostrum, however, had only about a 5% reduction in their villus height (p < 0.001 compared to control animals receiving indomethacin). This shows that colostrum prevents damage from occurring to gastrointestinal epithelial cells.

### In vitro studies

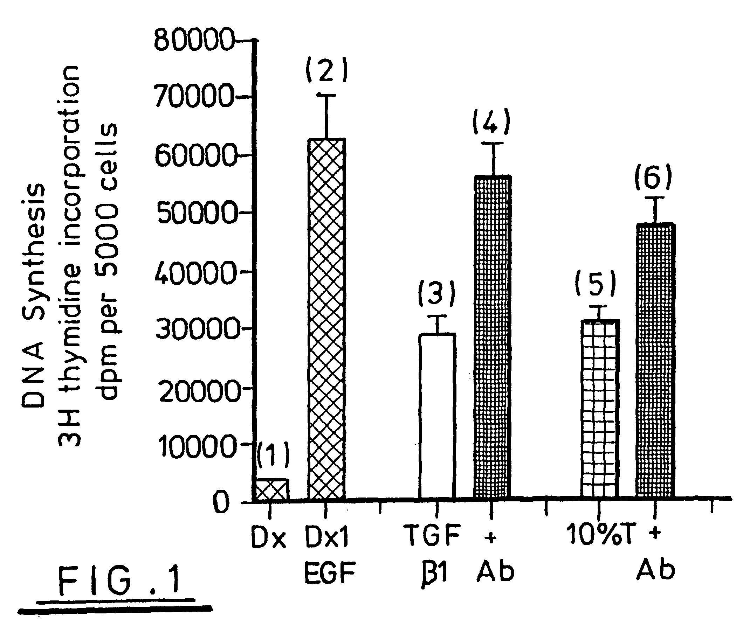

The addition to hepatocytes of increasing doses of EGF caused a dose-dependent increase in ³H thymidine incorporation (an index of the rate of hepatocyte proliferation). Referring to Fig. 1. a control response of 10 mg/ml of EGF increased ³H thymidine incorporation from a basal of approximately 3.000 DPM ( 1 ) to about 63,000 DPM (2). The co-administration of 0.1 ng/ml TGFβ with 10mg/ml EGF reduced ³H thymidine incorporation to about 50% (3) suggesting that TGFβ inhibits cell proliferation. This effect was removed when TGFβ neutralising antibody (referred in Fig.1 as Ab for convenience) was also added to the system (4). Significant reductions in ³H thymidine uptake caused by EGF were also seen with 10% colostrum (P < 0.01 vs. cells stimulated with EGF alone), the vast majority of this effect was removed when the TGFβ neutralising antibody was also present (5).

The *in vitro* and *in vivo* models demonstrate the value of colostral growth factors in preventing gastrointestinal damage. Addition of colostrum, but not milk, to the drinking water of mice markedly reduced the amount of small intestinal injury caused by indomethacin. The *in vitro* studies show that the predominant biological effect of colostrum preparations is a growth inhibitory response which can be reversed by the addition of a TGFβ neutralising antibody. This indicates that TGFβ plays an essential role in the effect of colostrum on epithelial cells.

### EXAMPLE 2

18 Male Sprague Dawley rats (225-250 g) were divided into three groups (six per group). The rats were placed in Bollman restraint cages. One group was given a continuous subcutaneous infusion of saline (control infusion), and the other two groups were given a continuous subcutaneous infusion of TGFβ1 at cither 0.15 or 1.5 µg/kg/h. Thirty minutes later all animals received 20 mg/kg of indomethacin (Sigma, UK) subcutaneously. Three hours after the injection of indomethacin, the animals were killed and their stomachs removed. The oesophageal opening was ligated, the stomachs inflated with 4 ml of formalin and the duodenal opening was then ligated to keep the stomachs in an inflated state. Stomachs were left overnight in formalin. The next day the stomachs were cut open along the greater curve and the total area of ulceration per stomach (mm²/stomach) was assessed using a dissecting microscope (X10) with the aid of a reference square grid.

Two additional groups (six rats per group) received a single 2 ml gavage of saline or TGFβ (0.5 µg per rat). Both the saline and TGFβ1 solutions also contained 2% hydroxymethylpropylcellulose to slow the rate of gastic emptying, allowing the TGFβ to remain in contact with the stomach for a greater period. Rats were then placed in the restraint cages. 30 min after the gavage, all rats received a single dose of indomethacin 20 mg/kg subcutaneously. Three hours after the administration of indomethacin animals were killed and assessed as above.

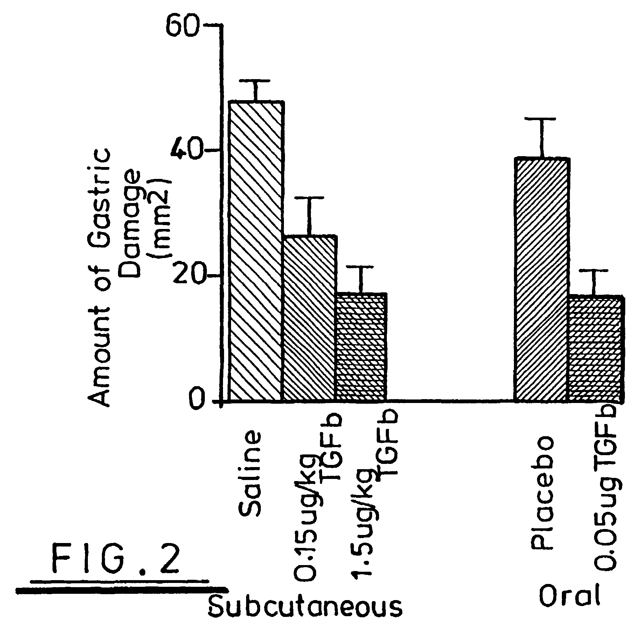

The results (mean) for each group are shown in Figure 2. As can be seen from this Figure. TGFβ administered by either route proved to be beneficial in decreasing the amount of injury seen three hours after indomethacine inhibition.

## Claims

1. The use of a composition containing Transforming Growth Factor β (TGF-β) for the manufacture of a medicament for prophylactic treatment of a gastrointestinal condition at least partially **characterised by** damage to epithelial cells, the condition being caused by administration of a non-steroidal anti-inflammatory drug.

2. The use according to claim 1 wherein the TGF-β is obtainable from colostrum.

3. The use according to claim 1 or claim 2 wherein from 0.05 to 2500µgrammes of TGF-β is to be administered daily.

4. The use according to claim 3 wherein from 25 to 1000µgrammes of TGF-β is to be administered daily.

5. The use according to claim 4 wherein from 50 to 500µgrammes of TGF-β is to be administered daily.

6. The use according to any preceding claim wherein the TGF-β is derived from naturally produced TGF-β isolated from an organism in which it was synthesised, TGF-β produced by cell culture or fermentation of TGF-β expressing cells, artificially synthesised TGF-β, modified TGF-β produced from genetically engineered organisms, compounds which activate TGF-β receptors (TGF-β receptor agonists), compounds which regulate TGF-β intracellular signalling and compounds which influence TGF-β synthesis or breakdown.

7. The use according to any one of claims 1 to 5 wherein the medicament comprises colostrum, or a derivative thereof, containing TGF-β.

8. The use according to claim 7 wherein the colostrum or derivative thereof is of bovine origin.

9. The use according to claim 7 or 8 wherein the colostrum is obtained in the first 48 hours post parturition.

10. The use according to claim 7 or 8 wherein the colostrum is obtained from the first and / or second milking post parturition.

11. The use according to any one of claims 7 to 10 wherein a colostrum derivative is used and is in a spraydried form.

12. The use according to any one of claims 7 to 11 wherein a colostrum derivative is used and is a liquid reconstituted from a spraydried colostrum derivative.

13. The use according to claim 11 or 12 wherein from 0.1 to 60.0 grammes of the spraydried colostrum derivative is to be administered daily.

14. The use according to any one of claims 7 to 10 wherein a colostrum derivative is used and that derivative is colostral whey or a colostral whey derivative.

15. The use according to claim 14 wherein the colostral whey derivative is obtained by ultrafiltration or microfiltration.

16. The use according to claims 14 or 15 wherein from 30 to 300mls of colostral whey or colostral whey derivative is to be administered daily.

17. The use according to any one of claims 7-16 wherein a colostrum derivative is used and that derivative is defatted.

18. The use according to any preceding claim wherein the medicament is to be administered by an enteral route.

19. The use according to claim 18 wherein the medicament is to be administered by an enema, , a nasogastric tube, gastrostomy tube or jejunostomy tube.

20. The use according to claim 18 or 19 wherein the medicament is to be administered in the form of a capsule or liquid.

21. The use according to claim 18 wherein the medicament is to be administered by an oral route.

22. The use according to claim 21 wherein the medicament is to be administered in the form of a tablet, capsule, liquid, food product or drink product.

23. The use according to claim 22 wherein the food product or drink product contains at least one of carbohydrates, a nitrogen source, vegetable oils, emulsifiers, oils containing long chain fatty acids, antioxidants, vitamins, soluble fibre, minerals and flavouring.

24. The use according to any preceding claim wherein the composition is to be administered from 6 to 72 hours in advance of the administration of the non-steroidal anti-inflammatory drug.

25. The use according to claim 24 wherein the composition is to be administered from 24 to 48 hours in advance of the administration of the non-steroidal anti-inflammatory drug.

26. The use according to any one of claims 1 to 25 wherein the non steroidal inflammatory drug is one of indomethacin, ibuprofen, azapropazone, naproxen, piroxicam, ketoprofen, diclofenac and asprin.

## Patentansprüche

1. Verwendung einer Zusammensetzung, enthaltend Transformationswachstumsfaktor β (TGF-β), für die Herstellung eines Arzneimittels für prophylaktische Behandlung einer gastrointestinalen- Krankheit mindestens teilweise **gekennzeichnet durch** Schädigung gegenüber Epithelzellen, wobei die Krankheit erzeugt wird **durch** Verabreichung eines nicht stereoiden entzündungshemmenden Arzneimittels.

2. Verwendung nach Anspruch 1, worin das TGF-β aus Colostrum erhältlich ist.

3. Verwendung nach Anspruch 1 oder Anspruch 2, worin 0,05 bis 2500 µg TGF-β täglich zu verabreichen sind.

4. Verwendung nach Anspruch 3, worin 25 bis 1000 µg TGF-β täglich zu verabreichen sind.

5. Verwendung nach Anspruch 4, worin 50 bis 500 µg TGF-β täglich zu verabreichen sind.

6. Verwendung nach einem vorhergehenden Anspruch, worin das TGF-β abstammt von natürlich hergestelltem TGF-β, isoliert von einem Organismus, in dem es synthetisiert wurde, TGF-β, hergestellt durch Zellkultur oder Fermentation von TGF-β exprimierenden Zellen, künstlich synthetisiertem TGF-β, modifiziertem TGF-β, hergestellt aus genetisch hergestellten Organismen, Verbindungen, die TGF-β Rezeptoren aktivieren (TGF-β Rezeptoragonisten), Verbindungen, die TGF-β intrazelluläres Signalbilden regulieren, und Verbindungen, die TGF-β Synthese oder Abbau beeinflussen.

7. Verwendung nach einem von Ansprüchen 1 bis 5, worin das Arzneimittel Colostrum oder ein Derivat davon, enthaltend TGF-β, umfaßt.

8. Verwendung nach Anspruch 7, worin das Colostrum oder Derivat davon von Rinderursprung ist.

9. Verwendung nach Anspruch 7 oder 8, worin das Colostrum in den ersten 48 Stunden nach Partus erhalten wird.

10. Verwendung nach Anspruch 7 oder 8, worin das Colostrum aus dem ersten und/oder zweiten Milchentziehen nach Partus erhalten wird.

11. Verwendung nach einem von Ansprüchen 7 bis 10, worin ein Colostrumderivat verwendet wird und in einer sprühgetrockneten Form ist.

12. Verwendung nach einem von Ansprüchen 7 bis 11, worin ein Colostrumderivat verwendet wird und eine Flüssigkeit, rekonstituiert aus einem sprühgetrockneten Colostrumderivat, ist.

13. Verwendung nach Anspruch 11 oder 12, worin 0,1 bis 60,0 Gramm des sprühgetrockneten Colostrumderivats täglich zu verabreichen sind.

14. Verwendung nach einem von Ansprüchen 7 bis 10, worin ein Colostrumderivat verwendet wird, und daß das Derivat Colostrummolke oder ein Colostrummolkederivat ist.

15. Verwendung nach Anspruch 14, worin das Colostrummolkederivat durch Ultrafiltration oder Mikrofiltration erhalten wird.

16. Verwendung nach Anspruch 14 oder 15, worin 30 bis 300 ml Colostrummolke oder Colostrummolkederivat täglich zu verabreichen sind.

17. Verwendung nach einem von Ansprüchen 7-16, worin ein Colostrumderivat verwendet wird und das Derivat entfettet ist.

18. Verwendung nach einem vorhergehenden Anspruch, worin das Arzneimittel durch einen enterischen Weg zu verabreichen ist.

19. Verwendung nach Anspruch 18, worin das Arzneimittel durch einen Einlauf, eine Nasensonde, Gastrostomieröhre oder Jejunostomieröhre zu verabreichen ist.

20. Verwendung nach Anspruch 18 oder 19, worin das Arzneimittel in der Form einer Kapsel oder Flüssigkeit zu verabreichen ist.

21. Verwendung nach Anspruch 18, worin das Arzneimittel auf einem oralen Weg zu verabreichen ist.

22. Verwendung nach Anspruch 21, worin das Arzneimittel in der Form einer Tablette, Kapsel, Flüssigkeit, Nahrungsmittelprodukt oder Trinkprodukt zu verabreichen ist.

23. Verwendung nach Anspruch 22, worin das Nahrungsmittelprodukt oder Trinkprodukt mindestens eines von Kohlehydraten, einer Stickstoffquelle, Pflanzenölen, Emulgatoren, Ölen, enthaltend langkettige Fettsäuren, Antioxidanzien, Vitaminen, lösliche Faser, Mineralien und Aromastoff enthält.

24. Verwendung nach einem vorhergehenden Anspruch, worin die Zusammensetzung 6 bis 72 Stunden vor der Verabreichung des nicht stereoiden entzündungshemmenden Arzneimittels zu verabreichen ist.

25. Verwendung nach Anspruch 24, worin die Zusammensetzung 24 Stunden bis 48 Stunden vor der Verabreichung des nicht stereoiden entzündungshemmenden Arzneimittels zu verabreichen ist.

26. Verwendung nach einem von Ansprüchen 1 bis 25, worin das nicht steroide entzündungshemmende Arzneimittel eines von Indomethacin, Ibuprofen, Azapropazon, Naproxen, Piroxicam, Ketoprofen, Diclofenac und Aspirin ist.

## Revendications

1. Utilisation d'une composition contenant du facteur de croissance transformant β (TGF-β) pour la fabrication d'un médicament pour le traitement prophylactique d'une affection gastro-intestinale au moins partiellement **caractérisée par** une lésion de cellules épithéliales, l'affection étant provoquée par l'administration d'une substance médicamenteuse anti-inflammatoire non stéroïdienne.

2. Utilisation selon la revendication 1, dans laquelle le TGF-β peut être obtenu à partir de colostrum.

3. Utilisation selon la revendication 1 ou la revendication 2, dans laquelle de 0,05 à 2500 µg de TGF-β doivent être administrés quotidiennement.

4. Utilisation selon la revendication 3, dans laquelle de 25 à 1000 µg de TGF-β doivent être administrés quotidiennement.

5. Utilisation selon la revendication 4, dans laquelle de 50 à 500 µg de TGF-β doivent être administrés quotidiennement.

6. Utilisation selon l'une quelconque des revendications précédentes, dans laquelle le TGF-β est obtenu à partir d'un TGF-β produit naturellement, isolé à partir d'un organisme dans lequel il a été synthétisé, d'un TGF-β produit par culture cellulaire ou fermentation de cellules exprimant le TGF-β, de TGF-β synthétisé artificiellement, de TGF-β modifié produit à partir d'organismes manipulés génétiquement, de composés qui activent les récepteurs de TGF-β (agonistes des récepteurs de TGF-β), de composés qui régulent la transmission des signaux intracellulaires du TGF-β et de composés qui influencent la synthèse ou la dégradation du TGF-β.

7. Utilisation selon l'une quelconque des revendications 1 à 5, dans laquelle le médicament comprend du colostrum, ou un dérivé de celui-ci, contenant du TGF-β.

8. Utilisation selon la revendication 7, dans laquelle le colostrum ou le dérivé de celui-ci est d'origine bovine.

9. Utilisation selon la revendication 7 ou 8, dans laquelle le colostrum est obtenu dans les 48 heures suivant la mise-bas.

10. Utilisation selon la revendication 7 ou 8, dans laquelle le colostrum est obtenu à partir de la première et/ou seconde traite après la mise-bas.

11. Utilisation selon l'une quelconque des revendications 7 à 10, dans laquelle un dérivé de colostrum est utilisé et est sous forme séchée par atomisation.

12. Utilisation selon l'une quelconque des revendications 7 à 11, dans laquelle un dérivé de colostrum est utilisé et est un liquide reconstitué à partir d'un dérivé de colostrum séché par atomisation.

13. Utilisation selon la revendication 11 ou 12, dans laquelle de 0,1 à 60,0 g du dérivé de colostrum séché par atomisation doivent être administrés quotidiennement.

14. Utilisation selon l'une quelconque des revendications 7 à 10, dans laquelle un dérivé de colostrum est utilisé et ce dérivé est du lactosérum colostral ou un dérivé de lactosérum colostral.

15. Utilisation selon la revendication 14, dans laquelle le dérivé de lactosérum colostral est obtenu par ultrafiltration ou microfiltration.

16. Utilisation selon les revendications 14 ou 15, dans laquelle de 30 à 300 ml de lactosérum colostral ou de dérivé de lactosérum colostral doivent être administrés quotidiennement.

17. Utilisation selon l'une quelconque des revendications 7 à 16, dans laquelle un dérivé de colostrum est utilisé et ce dérivé est dégraissé.

18. Utilisation selon l'une quelconque des revendications précédentes, dans laquelle le médicament doit être administré par une voie entérale.

19. Utilisation selon la revendication 18, dans laquelle le médicament doit être administré par un lavement, un tube nasogastrique, un tube de gastrostomie ou un tube de jéjunostomie.

20. Utilisation selon la revendication 18 ou 19, dans laquelle le médicament doit être administré sous la forme d'une capsule ou d'un liquide.

21. Utilisation selon la revendication 18, dans laquelle le médicament doit être administré par une voie orale.

22. Utilisation selon la revendication 21, dans laquelle le médicament doit être administré sous la forme d'un comprimé, d'une capsule, d'un liquide, d'un produit alimentaire ou d'un produit de boisson.

23. Utilisation selon la revendication 22, dans laquelle le produit alimentaire ou le produit de boisson contient au moins un ingrédient parmi les glucides, une source d'azote, les huiles végétales, les émulsifiants, les huiles contenant des acides gras à chaîne longue, les antioxydants, les vitamines, une fibre soluble, les minéraux et un aromatisant.

24. Utilisation selon l'une quelconque des revendications précédentes, dans laquelle la composition doit être administrée de 6 à 72 heures avant l'administration de la substance médicamenteuse anti-inflammatoire non stéroïdienne.

25. Utilisation selon la revendication 24, dans laquelle la composition doit être administrée de 24 à 48 heures avant l'administration de la substance médicamenteuse anti-inflammatoire non stéroïdienne.

26. Utilisation selon l'une quelconque des revendications 1 à 25, dans laquelle la substance médicamenteuse anti-inflammatoire non stéroïdienne est l'une parmi l'indométhacine, l'ibuprofène, l'azapropazone, le naproxène, le piroxicam, le kétoprofène, le diclofénac et l'aspirine.
